# EUROPEAN PATENT APPLICATION

(11) **EP 4 369 345 A1**
(43) Date of publication of application: **15.05.2024**
(21) Application number: 22207140.9
(22) Date of filing: 14.11.2022
(51) Int. Cl.: G16B 40/10

(54) **SYSTEM AND METHOD FOR OPTIMIZING ANALYSIS OF DIA DATA BY COMBINING SPECTRUM-CENTRIC WITH PEPTIDE-CENTRIC ANALYSIS**

(71) Applicant: Biognosys AG, 8952 Schlieren (CH)
(72) Inventor: GANDHI, Tejas Paresh, 5600 Lenzburg (CH); REITER, Lukas, 8800 Thalwil (CH); BERNHARDT, Oliver, 8902 Urdorf (CH)
(74) Representative: Bremi, Tobias Hans

(57) **Abstract**

A method for performing library-free search analysis comprising:
performing a search using a spectrum-centric approach (404) for a data (402);
performing at least one, preferably both of
a calibration (406) by using results from said spectrum-centric approach;
creating an optimized predicted library (409) by refining static prediction models (408a) by using the results of said spectrum-centric approach (404);

using said calibration (406) and/or said optimized predicted library (409) to initiate a peptide-centric search (411) for said data (402) based on an in-silico library (409);
creating a curated library (412) by combining the results of the spectrum-centric approach (404) with the results from the peptide-centric approach (412); and
analyzing the results of the curated library (412) using a second, preferably quantitative, peptide-centric search (413) of the data (402)

## Description

### TECHNICAL FIELD

The present invention relates to the analysis of compounds in mass spectrometry and more particularly to instruments, and methods for polypeptide analysis.

### PRIOR ART

Liquid chromatography coupled to Mass Spectrometry (LC-MS) has now been used for many years in the proteomic community for the identification and quantification of peptides (and thus proteins) from complex sample mixtures. In proteomics, the analytes are typically peptides generated by tryptic digestion of protein samples. The commonly most used approaches are variants of the so called LC-MS/MS or "shotgun" MS approach that is based on the generation of fragment ions from precursor ions that are automatically selected based on the precursor ion profiles (data dependent analysis, DDA). A main shortcoming of these methods is poor reproducibility which results in only partially overlapping protein sets in repeated analysis of substantially similar samples. Several new approaches have recently been developed that address these limitations and which can conceptually be described as targeted proteomics approaches.

The most mature technology is called selected Reaction Monitoring (SRM), frequently also referred to as multiple reaction monitoring (MRM). The targets for MRM experiments are defined on a rational basis and depend on the hypothesis to be tested in the experiment. Selected combinations of precursor ions and fragment ions (so called transitions, the set of transitions for one target precursor is called MRM assays) for these targets are programmed into a mass spectrometer, which then generates measurement data only for the defined targets.

Another variant of targeted proteomics is data independent acquisition (DIA). Here, the targeted aspect is introduced only on the data analysis level. Contrary to MRM, this approach does not require any preliminary method design prior to the sample injection. Since the LC-MS acquisition covers the complete analyte contents of a sample through the entire mass and retention time (RT) ranges the data can be mined a posteriori for any peptide/precursor of interest. Data is acquired in a data independent manner, on the complete mass range (e.g. 200-2000 Thomson) and through the entire chromatography, disregarding of the content of the sample. This is commonly achieved by stepping the selection window of the mass analyzer step by step through the complete mass range. In effect, this data acquisition method generates a complete fragment ion map for all the analytes present in the sample and relates the fragment ion spectra back to the precursor ion selection window in which the fragment ion spectra were acquired. This is achieved by widening the precursor isolation windows on the mass analyzer and thus accounting a priori for multiple precursors co-eluting and concomitantly participating to the fragmentation pattern recorded during the analysis. Such a precursor window is called a precursor selection window. The result is complex fragment ion spectra from multiple precursor fragmentations, that require a more challenging data analysis.

Unlike in shotgun proteomics, for the MRM and DIA technology spectra are repeatedly recorded for the same analytes with a high time resolution. The high time resolution when compared to shotgun proteomics, together with the limited fragment ion information for MRM and the limited fragment ion to precursor ion association for DIA, makes a completely new type of data analysis necessary. Since only a limited number of pre-defined analytes are being monitored, it is not necessary to make a shotgun proteomics type database search by comparing the spectra to a complete theoretical proteome. Instead, a number of scores have been described that are based on signal features such as shape, co-elution of transitions, and similarity of transition intensities to assay libraries.

In addition to the DIA methods mentioned above, a novel targeted proteomics technique was developed which can be considered a successor of SRM. This method, called parallel reaction monitoring (PRM), relies on a quadrupole mass filter which is combined with a high resolution mass analyzer, such as e.g. in a quadrupole-equipped bench-top orbitrap MS instrument. Replacing the last quadrupole of a triple quadrupole with a high resolution mass analyzer allows the parallel detection of all fragment ions at once. In principle it would also be possible to combine a linear ion trap with the orbitrap instead of the quadrupole. The advantage of PRM over SRM is that less prior knowledge about the target molecules is required. In terms of dynamic range PRM performs even better than SRM under some conditions due to its high selectivity.

A further development of this technique is multiplexed parallel reaction monitoring (mPRM) wherein not only single precursors are fragmented. In this method fragment ion spectra containing fragment ions from several precursors are created by either fragmenting larger m/z ranges or by multiplexing, which is sequentially fragmenting several precursors, and storing their fragment ions together for later measurement. In a further development internal standard triggered-parallel reaction monitoring (IS-PRM) has been proposed. In this method internal standard peptides are added to the sample. Based on their detection in a fast, low-resolution "watch" mode the acquisition parameters are switched to "quantitation" mode to ensure acquisition of endogenous peptides. This dynamic data acquisition minimizes the number of uninformative scans and can be applied to a variety of biological samples.

In proteomics experiments peptide levels in a sample are often determined relative to a labelled standard. Especially, isotopic labelling in combination with DDA and SRM mass spectrometry has proven useful to address a wide range of biological questions. In one exemplary setup, a sample containing endogenous, unlabeled, "light" peptides in unknown amounts is mixed with known quantities of synthetic, isotopically labelled, "heavy" peptides. During mass spectrometry analysis of the mixture, the mass difference introduced by the isotopic labels allows to distinguish the light endogenous from the heavy synthetic peptides in the sample and allows for their separate quantification.

Such experiments have proven so successful that pools of heavy-labelled synthetic peptides are now readily available from several commercial vendors. Alternatively, heavy-labelled peptide pools can also be produced via metabolically labelling proteins with heavy amino acids, or directly with heavy elemental isotopes, during in vitro or in vivo expression, and digesting said protein to peptides. The advantage of synthesizing peptides is that it is much faster and purification as well as absolute quantification of synthesized peptides is easier. Furthermore, incorporating only one heavy-labelled amino acid, rather than heavy elemental isotopes such as 15N for the whole peptide, has the advantage of producing a constant mass shift.

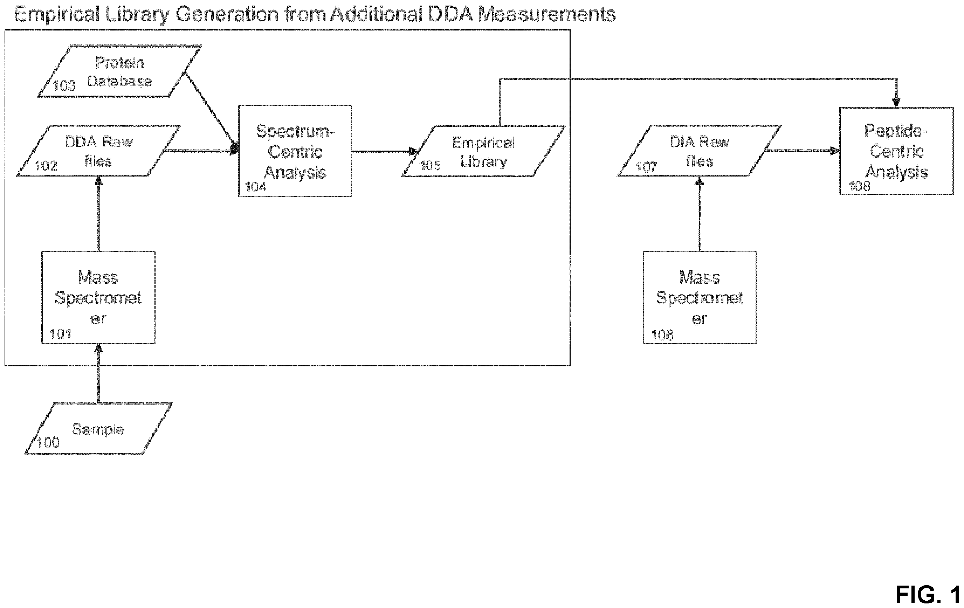

Analysis of Data Independent Acquisition (DIA) 108, see **Fig. 1**, typically relies on a spectral library 105 that describes the peptides belonging to a protein in terms of m/z, retention time, ion mobility, charge, and expected fragmentation spectra. Accurate description of the peptide facilitates deconvolution of a typical complex DIA MS2 spectrum which can be a product of tens of peptides. It has been previously shown that empirical spectral library-based DIA analysis can achieve unparalleled depth of proteome coverage. However, creating a good empirical library is time consuming and costly as it requires acquiring additional measurements 101.

**Fig. 1** is an illustration of a classical workflow for DIA analysis. In a classical workflow for DIA analysis, samples are first measured by a mass spectrometer 101 supplied with a sample 100 in a data dependent acquisition mode 102. These measurements are typically searched against a theoretical protein database 103 in a spectrum-centric analysis 104 to create an empirical library 105. Then the sample is re-measured by a mass spectrometer 106 in a data independent acquisition mode 107. The DIA data is searched against the previously generated empirical library 105 in a peptide-centric way 108 to get the final quantitative results.

Therefore, being able to process DIA data without the need to acquire library specific measurements would greatly benefit the field. Workflows that allow processing of DIA-data by creating a library without acquiring library specific measurements are commonly known as "library-free" DIA analysis.

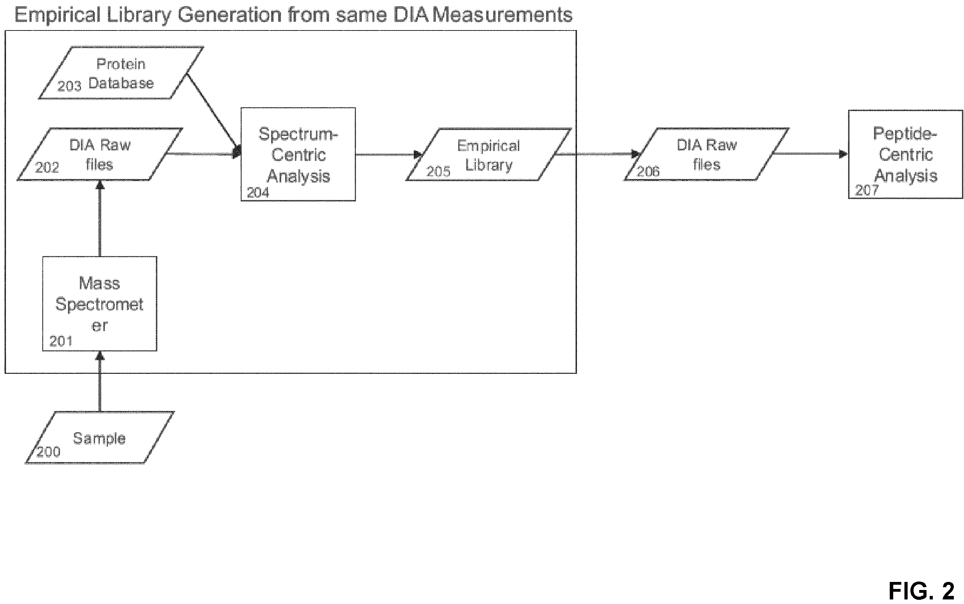

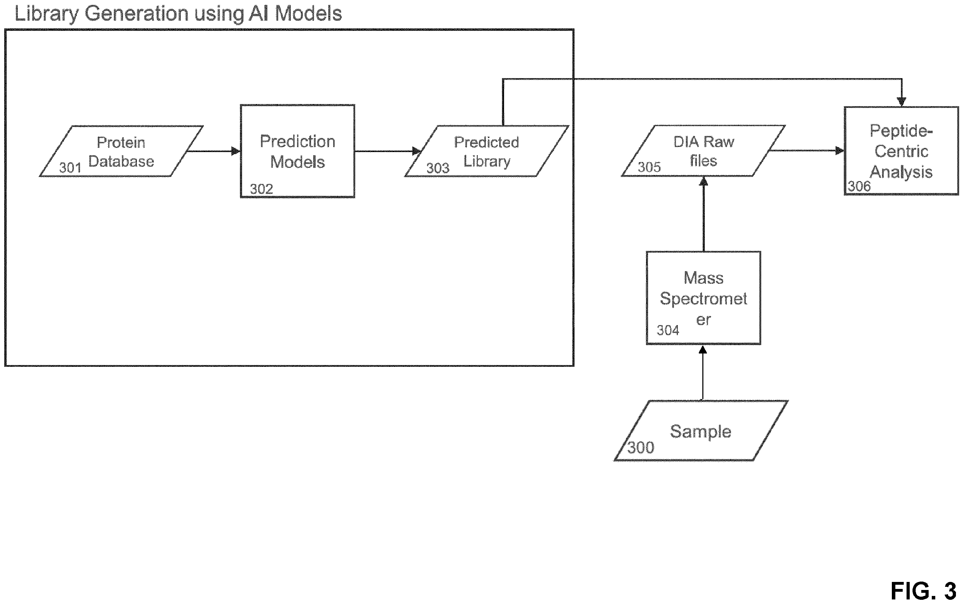

There have been two different library-free workflows proposed in the past as illustrated by **Fig. 2** and **Fig. 3****.**

In the first workflow according to **Fig. 2**, samples are first measured by a mass spectrometer 101 supplied with a sample 200 in a data independent acquisition mode 202. DIA data 202 is analyzed directly in a spectrum-centric approach 204 using a protein database 203. Optionally, and in a second step, those results can be used to build an empirical library 205 which is then used to re-analyze the same DIA data 206 in a peptide-centric approach 207 which typically yields a better quantification. While this workflow works well in all cases, it requires a good MS1 signal for deconvolution of complex MS2 spectra in DIA. This means that it often does not reach the same depth of coverage as an empirical library.

**Fig. 2** is an illustration of library free workflow with spectrum centric analysis. In a spectrum-centric based library free workflow, the samples are only measured once by a mass spectrometer 201 in data independent acquisition mode 202. These measurements are searched against a protein database 203 in a spectrum-centric manner 204 to create an empirical library 205. The library is then used to re-analyze the same DIA raw files 206 in a peptide-centric analysis 207 to get the final quantitative results.

The second library-free workflow according to **Fig. 3** creates a spectral library using AI assisted prediction models 302 for peptide characteristics to predict a proteome level library and then perform peptide-centric analysis of the DIA data 305 using this library. This approach does not rely on strong MS1 signals but suffers from high computational demand as every peptide in the library is queried in the data. The unspecific nature of the predicted libraries can make the peptide-centric analysis of DIA data 306 less robust and slow because of various reasons. This can be due to difficulty in deriving parameters optimized for an experiment and loss of sensitivity. Therefore, in-silico libraries often do not perform as well as empirical libraries in terms of depth of proteome coverage and data completeness.

**Fig. 3** is an illustration of such a library-free workflow with predicted library, as discussed previously. In a predicted library-based library free workflow, a predicted library 303 is created using a protein database 301 and typically artificial intelligence-based prediction models 302. Samples 300 are measured by a mass spectrometer 304 only once in data independent acquisition mode 305. The measurements 305 are searched against the predicted library 303 in a peptide centric analysis 306 to get the final quantitative results. Therefore, a system and method are needed that can achieve the library-free workflow wherein the disadvantages of library-free workflows are minimized.

### SUMMARY OF THE INVENTION

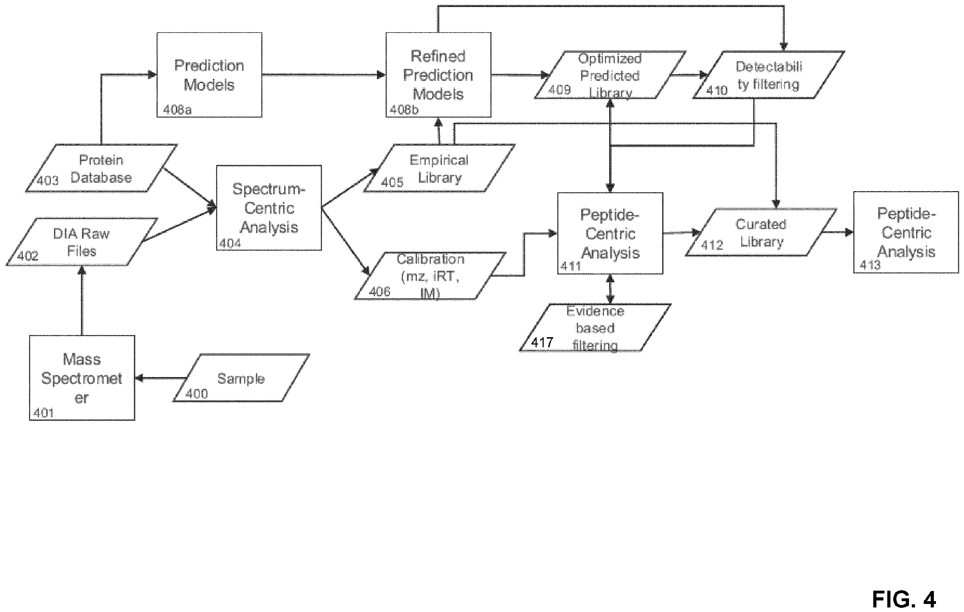

The present implementation is a significant improvement on the aforementioned prior-art. The present invention describes a new workflow (schematically exemplified in **Fig. 4**) that improves "library-free" analysis of DIA by combining the strengths of spectrum-centric analysis (schematically exemplified in **Fig. 2**) with in-silico predicted libraries (schematically exemplified in **Fig. 3**) in a novel manner.

The present invention solves the challenge of calibration as well as the problem of computation with a novel peptide-centric analysis.

The present invention relates to a method as defined in claim 1 and as further specified in the respective dependent claims.

### Definitions:

**LC-MS/MS:** Tandem mass spectrometry coupled to a liquid chromatography system, a technique in instrumental analysis where one or more mass analyzers are coupled together behind a liquid chromatography system using an additional reaction step to increase their abilities to analyse chemical samples.

**MS1, MS2:** The molecules of a given sample in an LC-MS/MS experiment are ionized and their mass-to-charge ratio (often given as m/z or m/Q) is measured/selected by the mass analyzer (designated MS1). Ions of a particular m/z-ratio coming from MS1 are selected and then made to split into smaller fragment ions, e.g. by collision-induced dissociation, ion-molecule reaction, or photo-dissociation. These fragments are then introduced into the mass analyzer (MS2), which in turn measures the fragments by their m/z-ratio. The fragmentation step makes it possible to identify and separate ionized molecules that have very similar m/z-ratios but produce different fragmentation patterns in MS2. The unfragmented peptide ion that dissociates to a smaller fragment ion, usually as a result of collision-induced dissociation in an MS/MS experiment, is typically referred to as precursor. **Data dependent acquisition (DDA)**: LC-MS/MS or "shotgun" MS approach that is based on the generation of fragment ions from precursor ions that are automatically selected in the first (MS1) dimension based on the precursor ion profiles in that dimension. The window for the second (MS2) dimension is chosen as a function MS1 output (single precursor peak) automatically by the machine. This means that in this mode the MS2 dimension is not continuously sampled but only selectively as a function of the MS1 signal. In a typical shotgun acquisition method, top 10 precursor ions are selected for fragmentation per MS1 scan by the MS for measurement in MS2 with a relatively narrow isolation width of 1-2 Thomson. Precursor ions that have been selected for fragmentation are also typically ignored by the MS in the subsequent scans to allow fragmentation of new precursor ions. **Data independent acquisition (DIA)**: LC-MS/MS approach, in this mode, all ionized compounds of a given sample that fall within a specified mass range in the first MS1 dimension are fragmented in a systematic and unbiased fashion resulting in corresponding spectra in the MS2 dimension. In contrast to DDA, in this case the MS2 space is continuously sampled. This not only leads to a larger data volume, but also has the effect that the spectra measured in the MS2 space comprise fragments not just from one precursor in the MS1 dimension but potentially from several such precursors. The common feature of DIA methods is that instead of selecting and sequencing a single precursor peak, wider m/z windows are fragmented resulting in complex spectra containing fragment ions of several precursors. This avoids the missing peptide ID data points typical for shotgun methods and potentially allows sequencing whole proteomes within one run, which offers a clear advantage over the small number of peptides that can be monitored per run by SRM. Furthermore, DIA have excellent sensitivity and a large dynamic range. To identify the peptides present in a sample, the fragment ion spectra can be searched against theoretical spectra or can be mined using SRM-like transitions. The detected fragments are subsequently arranged in SRM-like peak groups. In DIA acquisition, windows size in MS2 dimension is often more than 30 Thomson. This means that a typical MS2 scan in DIA is more complex than in DDA because of significantly more precursor ions being co-fragmented.

**Protein database**: a database, preferably selectively just for the organism of which the sample originates, comprising peptide and protein data from that organism, which means sequence information but no spectral information.

**Spectral library**: a database which contains information about peptide and protein systems as well as about fragments thereof, and which specifically associates to these peptides, proteins and fragments spectral information from an LC-MS/MS experiment, including (indexed) retention time, ion mobility, m/z ratios and expected fragment ion relative intensities.

**Empirical (spectral) library**: is a spectral library obtained based on an LC-MS/MS experiment typically using DDA and analysis of the data using a protein database and a spectrum-centric analysis.

**In-silico spectral library**: is a spectral library obtained using computer simulation results, such as artificial intelligence/deep learning algorithms. This type of library is also called predicted (spectral) library.

**Optimized predicted library**: is a predicted library that is created using static prediction models which are further refined using empirical data of an experiment.

**Static prediction model**: is a prediction model that can be created using optimization algorithms including using a deep neural network which is trained on a well-defined training data. This model can be used to infer on new data it has never seen without any further learning. We refer to such prediction models as static prediction models.

**Curated library**: a curated library refers to a library that is created by combining an empirical library from a spectrum centric analysis with the results of a peptide centric analysis based on a predicted or optimized predicted library. The combining is done by creating a consensus precursor for each precursor that were identified in both processes, so that in the curated library there is only one instance of each unique precursor. The consensus precursor summarizes the iRT, ion mobility, and observed fragments. **Deconvolution**: The process of resolving complex MS2 spectra to determine the underlying precursors that made up those spectra.

**Calibration**: Calibration is used to detect shifts between a theoretical quantity and its empirical counterpart and is a process of reducing the influence thereof. For example, an untuned MS can lead to a relatively large shift in the measured m/z in MS2 of precursors. A calibration step detects this shift and corrects for it usually based on some of a regression analysis. Calibration is typically done for m/z, ion mobility, and retention time (or iRT). **Library-free search analysis**: In the field, library-free search analysis of DIA refers to the process where you do not need to acquire MS measurements for the specific purpose of creating a spectral library only.

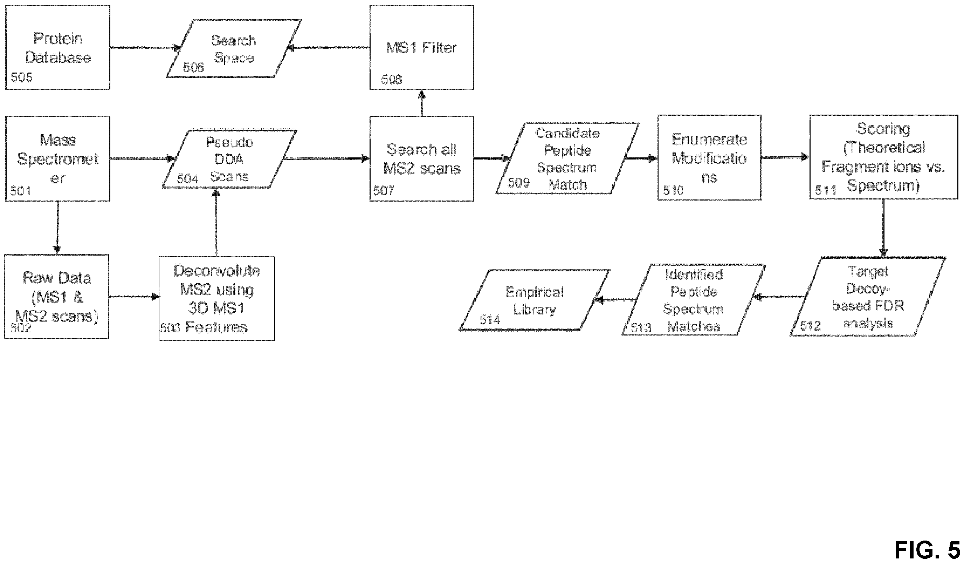

**Spectrum-centric analysis**: data analysis of data obtained in an LC-MS/MS experiment, which can be DDA or DIA data, in which the search is spectrum centric. This means that the spectra in the MS2 dimension are scanned for possible matches with all theoretical peptides and their fragments derived from a protein database typically with no or limited prior spectral information. Typically, the parent precursor ion for a MS2 spectrum is matched with a certain m/z tolerance to the theoretical m/z 508 (see also **Fig. 5** and the corresponding description further below) for all precursors in the search space 506 giving a set of candidate peptides 509. Then the candidate peptide which best explains the spectra in terms of theoretical fragment ions 511 is considered as the peptide spectrum match (PSM). No further prior information on the fragments is required.

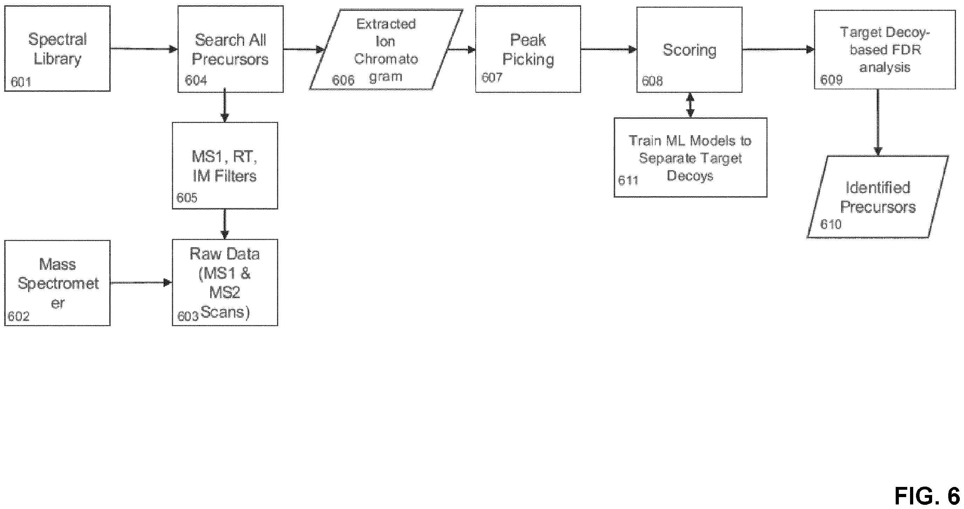

**Peptide-centric analysis/peptide centric search**: data analysis of data obtained in an LC-MS/MS experiment, which can be DDA or DIA data, in which the search is precursor centric. The predicted possible peptides and their fragments derived from a predicted spectral library or an empirical spectral library 601 (see also **Fig. 6** and the corresponding description further below) are queried against the spectra in the MS1 and MS2 dimension 604. In this analysis, spectral information of the peptides is required, in particular retention time, ion mobility, and likely to be observed fragment ions with relative fragment intensities. This information is used to narrow the search space of the peptide by querying only the spectrum that falls within a certain m/z, iRT or IM tolerance 605 and for scoring of matches. Having this additional information greatly improves the sensitivity of the analysis by leading to more powerful scores 608.

The present invention leverages the fact that spectrum-centric analysis (as schematically illustrated in **Fig. 2**) can reliably identify a significant portion of the sample also when working with a wider tolerance prior to calibration, i.e. with DIA data. Therefore, this approach can be used for obtaining empirical estimates of several important experiment level parameters along with peptide characteristics. This will in turn improve the performance of the in-silico predicted libraries instead of having that as a sole starting point for the data analysis (as schematically illustrated in **Fig. 3**). Furthermore, the present invention utilizes a simplified indexing concept of the spectrum-centric analysis to speed up the peptide-centric analysis. A person with ordinary skill in the art will understand that in addition to proteomics, this workflow can also be applicable to other mass spectrometer-based omics data, including but not limited to metabolomics.

The present invention overcomes the disadvantages of the prior art, as previously discussed. Typically, in a DIA analysis, tolerance parameters are estimated empirically by performing a pre-analysis. This is done based on a random subset of the spectral library (typically 10% of the library). This strategy works well when working with empirical libraries as they tend to be smaller than predicted libraries (by 10 to 100 fold) and highly specific to the underlying sample as it is derived from measuring the sample.

Unfortunately, trying this strategy with a predicted library does not work well or not work at all, because it will be highly unspecific. As such, we propose a novel workflow where a spectrum-centric analysis with a protein database is performed first. This step can provide calibrations in m/z, iRT (for retention time), and ion mobility dimensions. Alternatively, the spectrum centric analysis can also define the set of peptides to perform the peptide-centric pre-analysis to create calibrations, instead of using a random subset of peptides from the in-silico library. These calibrations across the different dimensions can then be used to extract ion chromatograms for each peptide in a predicted library with optimized tolerance windows around the predicted value for that dimension during the peptide-centric analysis. Importantly, the identified set of precursors coming from the spectrum centric analysis can be used in any parts of the peptide centric analysis where a random subset of peptides is generally used for optimizing parameters (such as training the machine learning model to best separate target from decoys 611).

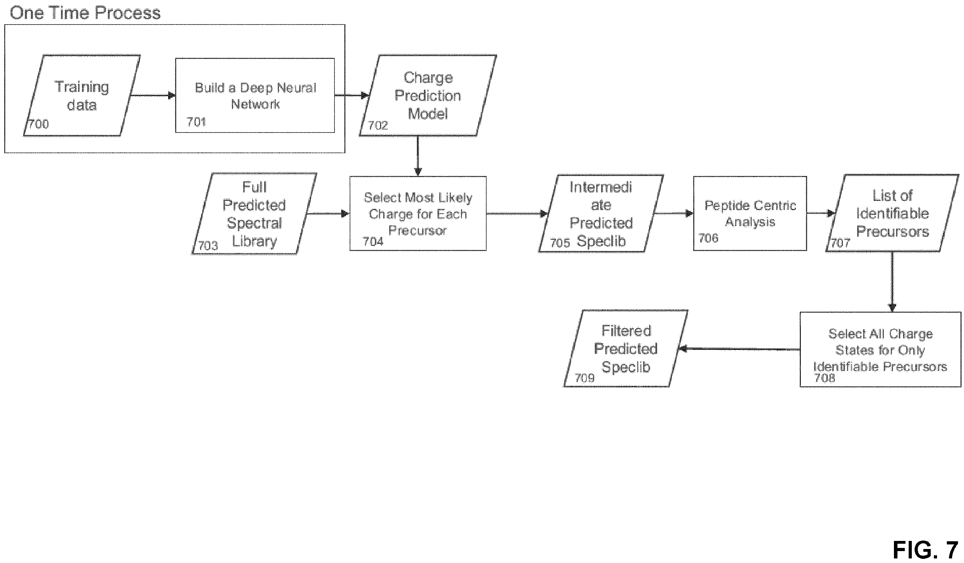

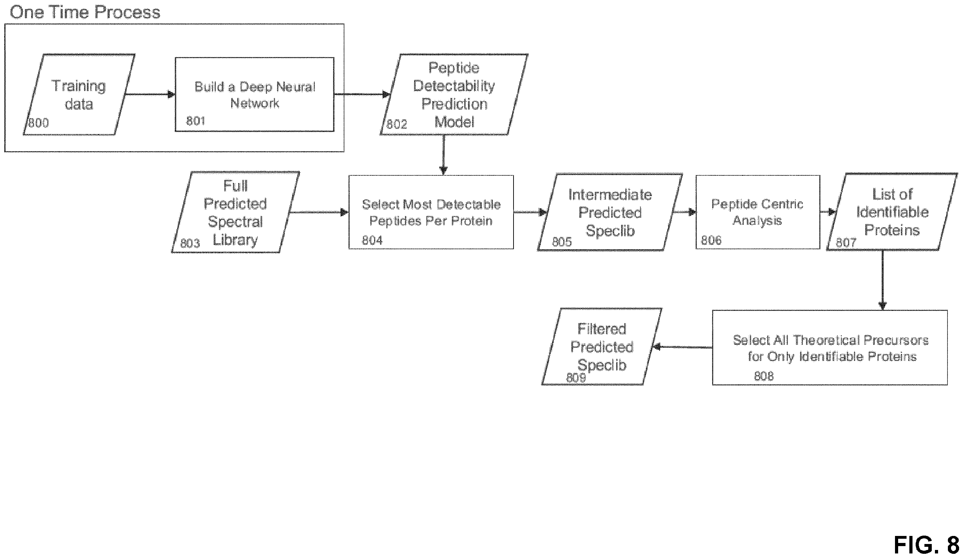

Since in-silico libraries can reach hundreds of millions of peptides in size, a method of prefiltering can help to reduce the number of peptides that are queried in the data with peptide-centric analysis. This can be done by checking for their presence in the raw data 402 within a predicted iRT and ion mobility range 407. In one embodiment, the present invention reduces the search space by using a prediction model (see also **Fig. 7** **and** **8** and the corresponding description further below) that can predict the detectability (e.g. likely charge state, proteotypicity, missed cleavage likelihood) of a peptide 410. A pre-analysis 411 is done to look for the most detectable version of a peptide and then in the second step expand the search space criteria to include related peptides of the identified peptides from the pre-analysis.

For example see **Fig. 7**, we created a deep neural network model that can predict the most likely charge state for a given peptide sequence 702. This model was trained using training data consisting of 1.2 million unique peptide sequences and their empirically observed charge state(s) 701. It allows to limit the search space in the analysis with the in-silico predicted library by only looking for the most likely charge stage of a peptide 704 instead of all possible charge states (typically charge 1 to 6). Then only for the precursor ions that are identifiable in its most likely charge state 707, the search space is expanded to include all possible charge states 708 (typically charge 1 to 6) to create a filtered spectral library 709. This concept can also be expanded to similar types of prediction models. For instance, if one can (accurately) rank most or all the theoretical peptide sequences for a protein by their detectability in a MS 802, then one can drastically narrow the search space by only looking at top 1 (or 3 or 6 or 10) observable peptides per protein in the first iteration 804. One can then expand the search space to create a filtered predicted spectral library 809 by including all theoretical precursors only for identifiable proteins 808. This kind of an iterative analysis coupled with powerful predictive models allows to drastically reduce the overall search space that one needs to tackle.

In one implementation, the empirically observed peptides via the spectrum-centric analysis can be used to refine the prediction models 408b. Prediction models based on AI are normally pre-developed on a specific set of training data 408a. The underlying training data can have systematics shifts from the empirical data if the data is measured with different parameters. It has been previously shown that prediction models can be refined "on-the-fly" via methods such as transfer learning to incorporate the peculiarities of the system for which it is currently trying to do the predictions. This means that prediction models can be refined for any peptide characteristics (e.g., ion mobility, retention time, compensation voltage, charge, missed cleavage, proteotypicity, etc.) on the results of the spectrum-centric analysis to improve the overall accuracy and precision of the predictions.

Next, the actual results from the spectrum-centric analysis can be used to improve identifications. Since the predicted libraries tend to be highly unspecific, it is beneficial to analyze them in two steps. The results of the first step are used to create a curated library by only keeping peptides identified with an identification threshold (e.g., create a new library with 10% or 1% false discovery threshold). In the main analysis, this curated library is then used. However, it is likely that some peptides will not make it in this library which were identifiable by the spectrum-centric analysis. So, it is beneficial to combine the results of the spectrum-centric analysis with the curated predicted library from the peptide-centric analysis to get an overall larger library for DIA analysis.

More specifically, in one aspect the present invention relates to a system and method for performing library-free search analysis that preferably combines the advantages of spectrum-centric search and in-silico library based search, comprising:
performing a search using a spectrum-centric approach for a data using a protein database, preferably data from data independent acquisition of a LC-MS/MS experiment on a sample, preferably on a digested sample of a mixture of proteins;
performing at least one, preferably both of
   a calibration by using results from said spectrum-centric approach;
   creating an optimized predicted library by refining static prediction models in the form of an in-silico spectral library, which is preferably based on the same protein database, by using the results of said spectrum-centric approach;
using said calibration and/or said optimized predicted library to initiate a peptide-centric search for said data based on said in-silico library;
creating a curated library by combining the results of the spectrum-centric approach with the results from the peptide-centric approach; and
analyzing the results of the curated library using a second peptide-centric approach of the data.

According to a preferred embodiment, the data is a set of data independent acquisition data obtained from a sample, preferably a digestive proteomic sample, in an LC-MS/MS experiment.

Calibration may typically comprise determination of at least one parameter associated with a respective peptide: mass to charge ratio, retention time, in particular indexed retention time, and ion mobility.

The optimized predicted library is typically obtained by generating an in-silico spectral library by numerical calculations from a protein database, and by generating an empirical library based on the data analysis of the spectrum centric approach, preferably by using the same protein database, and by comparing datasets in the in-silico spectral library with datasets in the empirical library for refinement of the parameters of the numerical calculations and for the generation of the optimized predicted library.

The optimized predicted library can further be subjected to a detectability filtering, preferably by numerical calculations based on the in-silico spectral library, and wherein the data after this detectability filtering is used in the curated library.

The results of the peptide centric analysis can further be filtered in an evidence-based filtering for final use of the data in the curated library. This can be achieved by the addition of an ion count-based pre-selection 608 before a peptide is subjected to a more extensive scoring process 609. Only if a sufficient amount of ions is present in the MS1 and MS2 spectra around the expected retention time, will a peptide be followed up on.

According to a further preferred embodiment, the optimised predicted library is further subjected to a detectability filtering, preferably by numerical calculations based on the in-silico spectral library, and wherein the data after this detectability filtering is used in the peptide centric search and/or in the curated library.

Preferably the detectability filtering is a charge based detectability filtering or peptide detectability based detectability filtering or a combination of the 2.

In case of a charge based detectability filtering, training data is used to predict a charge prediction model, and in addition using a predicted spectral library most likely charges for each precursor are determined, an intermediate predicted spectral library is generated and used for the peptide centric analysis, leading to a list of identifiable precursors, from which all charge states for only identifiable precursors are selected leading to a filtered predicted spectral library.

In case of a peptide detectability based detectability filtering, training data is used to predict a peptide detectability prediction model, and in addition using a predicted spectral library most likely detectable peptides per protein are determined, an intermediate predicted spectral library is generated and used for the peptide centric analysis, leading to a list of identifiable precursors, from which all theoretical precursors for only identifiable proteins are selected leading to a filtered predicted spectral library.

The results of the peptide centric analysis are preferably filtered in an evidence-based filtering for final use of the data in the curated library, wherein preferably the evidence-based filtering is an ion count based empirical filtering.

Preferably, results of a peptide centric analysis are filtered in an evidence-based filtering for final use of the data in the curated library in that ion chromatograms are extracted for each precursor based on tolerances, preferably in terms of at least one of iRT, IM, and m/z, and for each extracted ion chromatogram, peak picking is performed leading to precursor peak candidates, and for each of the precursor peak candidates, a spectrum centric score is calculated based on how many of the fragment ions and precursor isotope ions match the MS2 and MS1 spectra respectively, and if none of the peak candidates passes a pre-specified threshold, then the precursor is dropped from further analysis.

Further preferably, at least one of the peptide centric search and the second peptide centric search is carried out by using information from a spectral library to analyse the data specifically for selected precursors only.

Calibration preferably comprises determination of at least one parameter associated with a respective fragment: mass to charge ratio, retention time, in particular indexed retention time, expected fragment ion relative intensities, and ion mobility, and wherein the data, which is a set of DIA data, is subjected to a spectrum centric analysis using a protein database, from which precursors are identified, and the parameters are adjusted by using a prediction model preferably based on the same protein database to generate a predicted library for the basis of the calibration.

Calibration may also comprise determination of at least one parameter associated with a respective fragment: mass to charge ratio, retention time, in particular indexed retention time, expected fragment ion relative intensities, and ion mobility, and wherein the data, which is a set of DIA data, is subjected to a spectrum centric analysis using a protein database, from which precursors are identified, and the parameters are adjusted by using a prediction model preferably based on the same protein database to generate a predicted library for the basis of the calibration but using a specific selection for the calibration and using that selection for the peptide centric analysis.

Normally, the data is in the form of the sample mass spectroscopic intensity data acquired as a function of mass to charge ratio (m/z), of retention time (RT) as well as of ion mobility (IM) determined using an LC tandem mass spectroscopy method, preferably selected from the group of LC-MRM or LC-DIA.

Typically, the data is a set of data independent acquisition data obtained from a sample in an LC-MS/MS experiment and wherein the sample is a complex mixture of at least one protein of interest and further proteins and/or other biomolecules in the form of a complex native biological matrix which has been digested prior to LC-MS/MS analysis.

Typically, the at least one protein of interest is a protein based exclusively on proteinogenic amino acids, or is based on proteinogenic amino acids and carries post-translational modifications.

Also, the present invention relates to a system, in particular an LC-MS system, suitable and adapted for performing a method as detailed above, in particular a system suitable and adapted for performing library-free search analysis that combines the advantages of spectrum-centric search and in-silico library based search, comprising:
performing a search using a spectrum-centric approach for a data;
performing at least one, preferably both of
a calibration by using results from said spectrum-centric approach;
creating an optimized predicted library by refining static prediction models by using the results of said spectrum-centric approach;
using said calibration and/or said optimized predicted library to initiate a peptide-centric search for said data based on an in-silico library;
creating a curated library by combining the results of the spectrum-centric approach with the results from the peptide-centric approach; and
analyzing the results of the curated library using a second, preferably quantitative, peptide-centric approach search of the data.

Preferably, such a system is suitable and adapted to carry out a method for performing library-free search analysis comprising:
performing a search using a spectrum-centric approach for a data;
performing at least one, preferably both of
a calibration by using results from said spectrum-centric approach;
creating an optimized predicted library by refining static prediction models by using the results of said spectrum-centric approach;
using said calibration and/or said optimized predicted library to initiate a peptide-centric search for said data based on an in-silico library;
creating a curated library by combining the results of the spectrum-centric approach with the results from the peptide-centric approach; and
analyzing the results of the curated library using a second, preferably quantitative, peptide-centric search of the data.

Further the present invention relates to the use of a method as described above for the determination of at least one of the composition of the sample including quantitative information about the constituents, or a medically relevant conformation of the constituents, for the determination or the influence of protein-based drugs, for the influence of drugs or other ligands on proteins, or for quality control of protein-based pharmaceutical preparations.

Furthermore, the invention relates to a computer program product to cause an LC-MS device to execute the steps of the method as described above.

Also it relates to a computer-readable medium having stored thereon such a computer program product.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: schematically shows the analysis of Data Independent Acquisition (DIA) in a classical workflow;
- Fig. 2: schematically shows the analysis of Data Independent Acquisition (DIA) in a spectrum-centric approach;
- Fig. 3: schematically shows the analysis of Data Independent Acquisition (DIA) using Al assisted prediction models;
- Fig. 4: schematically shows an illustration of a new library-free workflow that combines peptide-centric and spectrum-centric strategies;
- Fig. 5: schematically shows a spectrum centric analysis workflow;
- Fig. 6: schematically shows a peptide centric analysis workflow;
- Fig. 7: schematically shows how charge-based detectability filtering can be applied to filter the large predicted spectral libraries in an iterative manner;
- Fig. 8: schematically shows how peptide detectability-based detectability filtering can be applied to filter the large predicted spectral libraries in an iterative manner

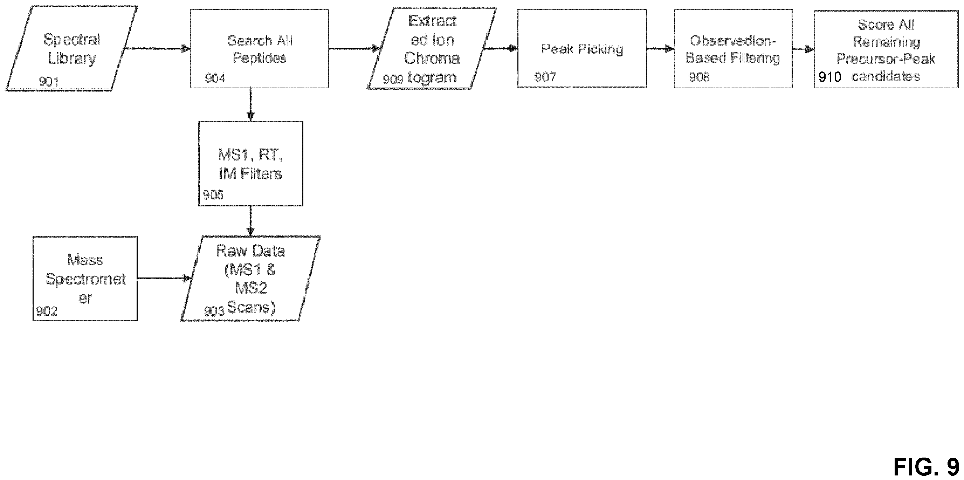

- Fig. 9: schematically shows how empirical based filtering works in peptide centric search using a predicted spectral library;
- Fig. 10a: schematically shows how results of the spectrum centric analysis can be used to create calibrations (m/z, iRT, and IM) as an input for the peptide centric analysis of predicted spectral libraries; and
- Figure 10b: schematically shows a similar but alternative method to one depicted in Fig 10a.

### DESCRIPTION OF PREFERRED EMBODIMENTS

The classical workflow for DIA analysis as illustrated in **Fig. 1** involves a first step of empirical library generation from additional DDA measurements. The corresponding process is well suited and works fine in the absence any advance spectral knowledge about the system but involves an additional measurement step in DDA mode for the actual establishment of the empirical library. So to generate the empirical library, which means to associate the MS2 spectra to the fragments, retention times, indexed retention times, ion mobility, an initial library generation step is required. This involves a two-step measurement process, which is costly and time-consuming.

The empirical library generation from the same DIA measurements as illustrated in **Fig. 2** is indeed possible, the problem however being that the data analyzed in the spectrum centric analysis 204 of the MS2 data is dealing with convoluted data, while if the basis is DDA measurements, the MS2 data are already filtered and not convoluted. This leads to a time-consuming analysis for the larger set of MS2 data, because this dimension is continuously scanned, but it also leads to less reliable results due to the mixed information in the MS2 data from several fragments. This has the effect that the analysis often misses out peptides and fragments, in the initially generated empirical library 205, and the second iteration with the DIA raw files 206 and using a peptide centric analysis 207 is necessary. Also the library free workflow with a predicted library as illustrated schematically in **Fig. 3** is possible, where the library generation is based on using computational models, for example AI or deep learning models. The prediction models 302 entail in-silico co-predictions of retention times, indexed retention times, ion mobility, fragments, et cetera. The problems associated with this approach are that the predicted library is very large also covering theoretical systems which cannot be detected. There is therefore a significantly larger amount of data for the MS2 data to be scanned against, and the less specific the library the worse a peptide centric analysis works. A further problem is associated with calibration. Calibration is used to eliminate systematics errors introduced by the instrumentation. Typically deliberation involves a first step a large threshold and a mini analysis is used to get the suitable and adapted thresholds, and then in a second step these are suitable and adapted thresholds are used for the actual analysis. In this respect reference is made to the methods as disclosed in WO 2022/184406, the content of which is incorporated into this disclosure as concerns the calibration.

According to that calibration method, using a database of reference peptide precursor data for retrieval of a region of interest for at least three reference peptide precursors in the mass to charge ratio (m/z), the retention time (RT) as well as in the ion mobility (IM) dimension, in a first step for at least three reference peptide precursors, preferably for all reference peptide precursors from the database of reference peptide precursor data, said sample mass spectroscopic intensity data is analyzed in the respective reference peptide precursor region of interest of mass to charge ratio (m/z), retention time (RT) as well as ion mobility (IM) dimension, and from that analysis empirically an adjusted center in the ion mobility dimension (IM) for each reference peptide precursor is determined and an ion mobility extraction width window in the ion mobility dimension (IM), preferably as a variable function of the ion mobility dimension (IM), is determined, and wherein in a second step for the identification of further peptide precursors from said sample mass spectroscopic intensity data, said empirically determined ion mobility extraction width window in the ion mobility dimension (IM), preferably as a variable function of the ion mobility dimension (IM) is used. The problem associated with the calibration is that due to the large number of elements in the predicted library there are too many elements to analyze, the analysis is therefore not only time-consuming but it is also not robust due to the huge number of hypothesis leading to a less stringent statistical analysis.

On the other hand this approach does not suffer the problem associated with selections based on the MS1 dimension, because any selection based on the MS1 dimension requires a sufficiently strong signal in that dimension, which is why the spectrum centric approach as illustrated in the context of **Fig. 2** leads to situations where one misses out systems due to insufficient sensitivity in the first dimension.

**Fig. 4** is an illustration of new library-free workflow that combines peptide-centric and spectrum-centric strategies. In the present invention, peptide-centric and spectrum-centric strategies are combined in a novel way. Samples, which typically are protein samples which have been subjected to digestion to produce shorter sub-sequences called peptides, are first separated in a liquid chromatography step, and then measured by a mass spectrometer 401 in a data independent acquisition mode 402. These measurements are searched against a protein database 403 in a spectrum-centric manner 404 to create an empirical library 405, and calibrations in m/z, iRT, and ion mobility dimensions 406.

The analysis of the spectrum centric step 404 takes two branches, one branch of determining the empirical spectral library 405, and one branch of determining the calibration 406.

The spectrum centric analysis of DIA data (**see** **Fig. 5**) involves deconvolution 503 of the complex MS2 spectra 502 obtained from a sample (not illustrated) in a spectrometer 501 due to co-fragmentation of multiple precursor ions. This deconvolution 503 is done by correlating the features in the MS1 scans over time with features in the MS2 spectra as one expects the fragment ions to peak similarly to the parent precursor ions. Pseudo-DDA like MS2 scans 504 are created based on this deconvolution which allows them to be searched in a spectrum centric manner 507 under MS1 filtering 508 against a search space 506 derived from a protein database 505. The empirical library 514 is created as a result of the precursor ions identified from this search 512. Typically, the parent precursor ion for a MS2 spectrum is matched with a certain m/z tolerance to the theoretical m/z in the MS1 filtering 508 for all precursors in the search space 506 giving a set of candidate peptides 509. Then the candidate peptide which best explains the spectra (using enumeration of modifications 510) in terms of theoretical fragment ions 511 is considered as the peptide spectrum match (PSM).

Next the false discovery rate (FDR) is calculated using a target decoy approach 512. In this step, a score threshold is selected in a way that only user-specified percentage of identifications will be false positive (typically 1%). All PSMs above this threshold are considered identified 513. All identified precursors are used to create an empirical spectral library 514 by creating consensus precursors which summarizes (e.g. averaging) observed peptide characteristics (iRT, IM, fragment intensities) in case the precursor was identified in multiple spectra and samples. This is either done by an average or weighted average or another statistical method, because in the library there is only one entry for each precursor, but one might identify that precursor multiple times (e.g. multiple DDA runs for the library) with slightly different measurement (fluctuations in RT, intensities due to noise, etc.).The empirical library is then an empirical consensus representation of each uniquely identified peptide precursor.

One of the key elements of the first branch is that information present in the empirical library 405 can be optionally used to refine existing prediction models for iRT, ion mobility, peptide fragmentation, peptide charge, peptide flyability, peptide cleavage and other characteristics 408a to create refined prediction models 408b. These refined prediction models with the protein database 403 are used to create an optimized predicted library 409. The predicted library 409 is then used to analyze the DIA raw files 402 in a peptide centric manner 411. So basically the empirical library 405 is used to tune the in-silico model, which means that for those systems effectively seen in the empirical library, the prediction parameters can be adapted, and the same prediction parameters can then be used for calculation of systems which are not seen in the empirical library.

The spectrum centric analysis is done only once for the determination of the empirical library. The precursors identified for each DIA measurement during the spectrum centric analysis are re-used to create a calibration in m/z, RT, and IM dimensions.

As for the second branch with the calibration 406, the advantage here is that from the analysis 404 it is known which precursor ions actually show up, and calibration can be carried out on the basis of these precursor ions and their spectral properties. This leads to a significant reduction in time, an improvement of sensitivity and robustness, and it should be noted that calibration, i.e. adapting the parameters to the specific machine situation and measuring parameters, often makes pure in silico approaches fail.

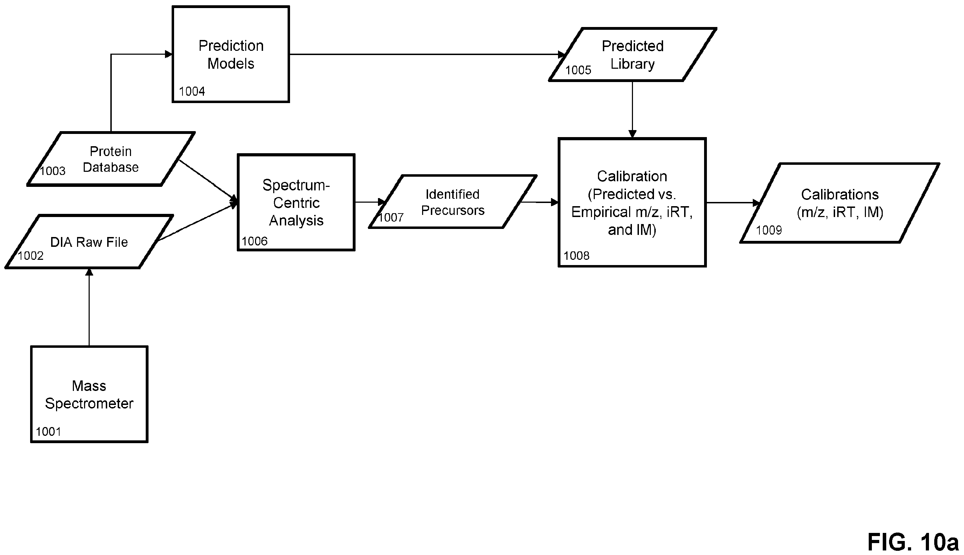

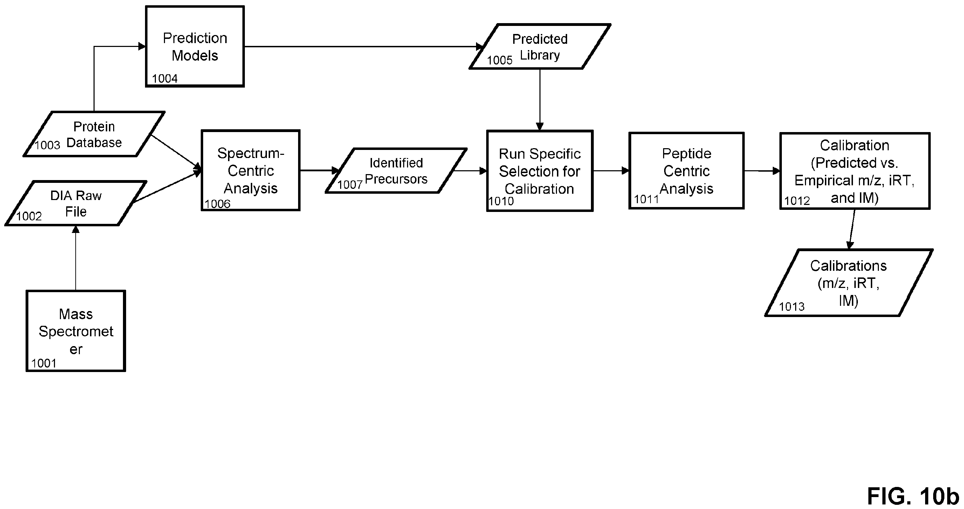

**Fig. 10a** **and** **10b** show two similar methods for calibration.

One can either (**see** **Fig. 10a**) create a run-specific calibration 1009 directly from the precursors 1007 identified by the spectrum centric analysis 1006 using a regression of predicted vs. empirical for m/z, iRT, and IM (1008).

Alternatively (**see** **Fig. 10b**), one can select precursors 1010 identified 1007 from the spectrum centric analysis 1006 of a run to perform a peptide centric analysis 1011 on that run. The results from this mini peptide centric analysis can be used to again create a regression of predicted vs. empirical for m/z, iRT, and IM (1013).

In both cases, the results from the spectrum centric analysis 1006 are the key components to make the calibration quick and robust.

In one implementation, evidence-based filtering of peptides 417 can be performed from the in-silico library based on their presence in the raw data 402 within predicted iRT and ion mobility tolerances 406. This can be achieved by using a fast spectrum centric score based on the MS1 and MS2 spectra 908 as a threshold before a precursor-peak candidate is subjected to a more extensive scoring process 910. Only if at least one precursor-peak candidate passes the threshold will a precursor be followed up on.

A typical peptide centric search of DIA data is illustrated in **Fig. 6****.** A sample (not illustrated) is analysed in a mass spectrometer 602 leading to the raw DIA data 603. A spectral library 601 is used for analyzing the DIA data. The spectral library can contain decoy precursors which are used for identification purposes later in the pipeline. Alternatively, decoys can be created on the fly from the expected or target precursors in the spectral library, for example, by reversing the sequences of the target precursors. A search 604 is carried out for all precursors (target and decoys) using filters 605. This results in an extracted ion chromatogram 606, in which the precursors are selected by peak picking 607 and scored using associated scoring 608. Machine learning model (ML) are trained to separate target from decoys 611 based on the subset of the data in an iterative manner. The trained machine learning model is then used to calculate a score for all precursors. This scoring is followed by target decoy-based false discovery rate (FDR) analysis 609 leading to the finally identified precursors 610. During the FDR analysis, the distribution of decoy precursors is used to calculate the threshold that will return precursors identified with a user-specified false discovery control (e.g. 1% FDR).

In another implementation, detectability filtering 410 can be performed based on predicted likelihood of observing a peptide, normally in a certain charge state or missed cleavage, to filter the predicted library. This can be done in an iterative manner whereby in a pre-analysis, only the most detectable peptides are searched, and then expanding the search space to all peptides related to the identified peptides from the pre-analysis.

A schematic illustration of charge base detectability filtering is illustrated in **Fig. 7****.** Training data 700 are used to build a deep neural network 701 and to generate a charge prediction model 702. Using the full predicted spectral library 703 this charge prediction model 702 is used to select the most likely charge for each precursor 704. This leads to an intermediate predicted spectral library 705. This is followed by a peptide centric analysis 706 based on raw DIA data, and finally leads to a list of identifiable precursors 707. Then all charge states but only for identifiable precursors are selected 708, leading to a filtered predicted spectral library 709.

The detectability filter can come in many forms of predictive models. For example, we created a deep neural network model that can predict the most likely charge state for a given peptide sequence 702. This model was trained using training data consisting of 1.2 million unique peptide sequences and their empirically observed charge state(s) 701. It allows to limit the search space in the analysis with the in-silico predicted library by only looking for the most likely charge stage of a peptide 704 instead of all possible charge states (typically charge 1 to 6). Then only for the precursor ions that are identifiable in its most likely charge state 707, we expand the search space to include all possible charge states 708 (typically charge 1 to 6) to create a filtered spectral library 709. This concept can also be expanded to similar types of prediction models.

For instance, as illustrated in **Fig. 8**, if one can accurately rank all the theoretical peptide sequences for a protein by their detectability in a MS 802, then one can drastically narrow the search space by only looking at top 1 (or 3 or 6 or 10) observable peptides per protein in the first iteration 804. Using training data 800 a deep neural network 801 is built to derive a peptide detectability prediction model 802. A full predicted spectral library 803 this peptide detectability prediction model 802 is used to select the most detectable peptides per protein 804. An intermediate predicted spectral library 805 is generated. This is followed by a peptide centric analysis 806 based on the raw DIA, and finally leads to a list of identifiable proteins 807. This is followed by selecting all theoretical precursors for only identifiable proteins 808, leading to a filtered predicted spectral library 809. One can thus expand the search space to create a filtered predicted spectral library 809 by including all theoretical precursors only for identifiable proteins 808. This kind of an iterative analysis coupled with powerful predictive models allows to drastically reduce the overall search space that one would need to tackle.

All the peptides that pass the filters 407, 410 or all the peptides in the optimized predicted library 409 are searched in a peptide-centric manner 411 based on the calibrations 406 that provide the shift from theoretical to empirical and tolerance threshold for each peptide in each dimension. Note that the peptide centric analysis 411 is not yet a quantitative analysis. The step 411 is only a fast identification and spectral properties determination step. Evidence-based filtering of peptides (**Fig**. **9**) is performed during the peptide centric analysis 904 of precursors in the predicted spectral library 901. Ion chromatograms are extracted for each precursor based on tolerances in iRT, IM, and m/z dimensions 905. Then for each extracted ion chromatogram (XIC, 909), peak picking is performed 907 which leads to precursor peak candidates. For each of the candidate precursor peak, a spectrum centric score is calculated based on how many of the fragment ions and precursor isotope ions match the MS2 and MS1 spectra respectively 908. If none of the peak candidates pass a pre-specified threshold, then the precursor is dropped from further analysis 910. This is important because it allows to efficiently deal with the large predicted spectral libraries.

A peptide centric analysis 411 is typically performed as illustrated in **Fig. 6** and as discussed above by querying all peptides of a given search-space / library 601 against the acquired data 604. The algorithm iterates over all peptides and extracts signals from the data corresponding to the peptides characteristics (like expected fragmentation, retention time and ion mobility) 605. Peak picking 607 is performed on the extracted signals and then the peaks are scored 608 against a set of scoring functions that focus on different signal characteristics. The peptides are then compared against artificially introduced false/random signals (decoys) to determine which peptides are statistically different from these random decoy signals.

Peptides identified with an FDR threshold by this analysis are used to create a new curated library which is also combined with the empirical library 405. If a precursor was observed in both libraries, then it is simply summarized by averaging its iRT, IM, and relative fragment intensities. Finally, this curated or combined library is used to search the DIA raw files again 402 in a peptide centric manner to get the final list of identified precursors. We then perform further post-processing steps such as quantification, normalization, post translational modification analysis, etc. based on these identifications to provide quantitative results with biological insights.A person with ordinary skill in the art will understand that the inventive method and system described in this disclosure can be applied to any intrinsic property of a peptide precursor ion that can be predicted beforehand. A person with ordinary skill in the art will also understand that the inventive method can be applied to similar work-flows and other implementations that comprise of similar components, even if they are arranged in a different manner.

While certain aspects of the present invention have been particularly shown and described with reference to exemplary embodiments thereof, it will be understood by those of ordinary skill in the art that various changes in form and details may be made therein without departing from the spirit and scope of the present invention as defined by the following claims. It will also be understood that the components of the present disclosure may comprise hardware components or a combination of hardware and software components. The hardware components, methods, and workflows may comprise any suitable tangible components that are structured or arranged to operate as described herein. Some of the hardware components may comprise processing circuitry (e.g., a processor or a group of processors) to perform the operations described herein. The software components may comprise code recorded on tangible computer-readable medium. The processing circuitry may be configured by the software components to perform the described operations. It is therefore desired that the present embodiments be considered in all respects as illustrative and not restrictive.

**LIST OF REFERENCE SIGNS**

| | | | |
|---|---|---|---|
| 101 | mass spectrometer | 405 | empirical spectral library |
| 102 | data dependent acquisition mode | 406 | calibrations |
| | | 407 | predicted iRT and ion mobility range |
| 103 | protein database | | |
| 104 | spectrum centric analysis | 410 | prediction of detectability of a peptide |
| 105 | spectral library | | |
| 106 | | 408a | training data |
| 107 | data Independent acquisition mode | 408b | prediction models |
| | | 409 | optimised predicted library |
| 108 | analysis of data Independent acquisition | 411 | pre-analysis, peptide centric analysis |
| 201 | mass spectrometer | 412 | curated library, sample specific library |
| 202 | mass spectrometer in data Independent acquisition mode | | |
| | | 413 | quantitative peptide centric analysis |
| 203 | protein database | 417 | evidence-based filtering501 mass spectrometer |
| 204 | spectrum centric approach | | |
| 205 | | 502 | raw data |
| 206 | DIA raw files | 503 | deconvolution |
| 207 | peptide centric approach | 504 | pseudo DDA scans |
| 301 | peptide database | 505 | protein database |
| 302 | artificial intelligence based prediction model | 506 | search space |
| | | 507 | searching MS2 scans |
| 303 | predicted library | 508 | MS filter |
| 304 | mass spectrometer | 509 | candidate peptide spectra match |
| 305 | data Independent acquisition mode | | |
| | | 510 | enumeration of modifications |
| 306 | peptide centric analysis line | 511 | scoring |
| 401 | mass spectrometer | 512 | target decoy based FDR |
| 402 | raw data, data independent acquisition mode | | analysis |
| | | 513 | identified peptide spectra matches |
| 403 | protein database | | |
| 404 | spectrum centric analysis | 514 | empirical library |
| 601 | spectral library | 806 | peptide centric analysis |
| 602 | mass spectrometer | 807 | list of identifiable proteins |
| 603 | raw data | 808 | selection of all theoretical precursors for only identifiable proteins |
| 604 | precursor search | | |
| 605 | filters | | |
| 606 | extracted ion chromatogram | 809 | filtered predicted spectral library |
| 607 | peak picking | | |
| 608 | scoring | 901 | predicted spectral library |
| 609 | target decoy based FDR analysis | 902 | mass spectrometer |
| | | 903 | raw data |
| 610 | identified precursors | 904 | searching all peptides |
| 611 | training with ML models to separate target decoys | 905 | filters |
| | | 907 | peak picking |
| 700 | training data | 908 | spectrum-centric scoring |
| 701 | deep neural network | 909 | extracted ion chromatogram |
| 702 | charge prediction model | 910 | score |
| 703 | full predicted spectral library | 1001 | mass spectrometer |
| 704 | selection of most likely charge for each precursor | 1002 | DIA raw data |
| | | 1003 | protein database |
| 705 | intermediate predicted spectral library | 1004 | prediction models |
| | | 1005 | predicted library |
| 706 | peptide centric analysis | 1006 | spectrum centric analysis |
| 707 | list of identifiable precursors | 1007 | identified precursors |
| 708 | selection of all charge states for only identical precursors | 1008 | calibration after spectrum centric analysis |
| 709 | filtered predicted spectral library | 1009 | calibrations after spectrum centric analysis |
| 800 | training data | 1010 | run specific selection for |
| 801 | deep neural network | | calibration |
| 802 | peptide detectability prediction model | 1011 | peptide centric analysis |
| | | 1012 | calibration after peptide centric analysis |
| 803 | full predicted spectral library | | |
| 804 | selection of most detectable peptides per protein | 1013 | calibrations after peptide centric analysis |
| 805 | intermediate predicted spectral library | | |
| | | DDA | data dependent acquisition |
| DIA | data Independent acquisition | | |
| FDR | false discovery rate | MS2 | second spectral dimension in LC-MS/MS experiment |
| IM | ion mobility | | |
| iRT | indexed retention time | RT | retention time |
| m/z | mass to charge ratio | SRM | Selected Reaction Monitoring |
| MRM | multiple reaction monitoring | XIC | extracted ion chromatogram |
| MS1 | first spectral dimension in LC-MS/MS experiment | | |

## Claims

1. A method for performing library-free search analysis comprising:
performing a search using a spectrum-centric approach (404) for a data (402);
performing at least one, preferably both of
a calibration (406) by using results from said spectrum-centric approach; creating an optimized predicted library (409) by refining static prediction models (408a) by using the results of said spectrum-centric approach (404);
using said calibration (406) and/or said optimized predicted library (409) to initiate a peptide-centric search (411) for said data (402) based on an in-silico library (409);
creating a curated library (412) by combining the results of the spectrum-centric approach (404) with the results from the peptide-centric approach (412); and
analyzing the results of the curated library (412) using a second, preferably quantitative, peptide-centric search (413) of the data (402).

2. The method according to claim 1, wherein the data (402) is a set of data independent acquisition data obtained from a sample (400), preferably a digestive proteomic sample, in an LC-MS/MS experiment.

3. The method according to any of the preceding claims, wherein calibration (406) comprises determination of at least one parameter associated with a respective fragment: mass to charge ratio, retention time, in particular indexed retention time, expected fragment ion relative intensities, and ion mobility.

4. The method according to any of the preceding claims, wherein the optimised predicted library (409) is obtained by generating an in-silico spectral library (408a) by numerical calculations from a protein database (403), and by generating an empirical library (405) based on the data analysis of the spectrum centric approach (404), preferably by using the same protein database (403), and by comparing datasets in the in-silico spectral library (408a) with datasets in the empirical library (405) for refinement of the parameters of the numerical calculations and for the generation of the optimised predicted library (409).

5. The method according to any of the preceding claims, wherein the optimised predicted library (409) is further subjected to a detectability filtering (410), preferably by numerical calculations based on the in-silico spectral library (408a), and wherein the data after this detectability filtering (410) is used in the peptide centric search (411) and/or in the curated library (412).

6. The method according to claim 5, wherein the detectability filtering is a charge based detectability filtering or peptide detectability based detectability filtering,
wherein in case of a charge based detectability filtering training data (700) is used to predict (701) a charge prediction model (702), and wherein in addition using a predicted spectral library (703) most likely charges for each precursor (704) are determined, an intermediate predicted spectral library (705) is generated and used for the peptide centric analysis (411, 706), leading to a list of identifiable precursors (707), from which all charge states for only identifiable precursors are selected (708) leading to a filtered predicted spectral library (709);
and wherein in case of a peptide detectability based detectability filtering training data (800) is used to predict (801) a peptide detectability prediction model (802), and wherein in addition using a predicted spectral library (803) most likely detectable peptides per protein (804) are determined, an intermediate predicted spectral library (805) is generated and used for the peptide centric analysis (411, 706), leading to a list of identifiable precursors (807), from which all theoretical precursors for only identifiable proteins are selected (808) leading to a filtered predicted spectral library (809).

7. The method according to any of the preceding claims, wherein the results of the peptide centric analysis (411) are filtered in an evidence-based filtering (407) for final use of the data in the curated library (412), wherein preferably the evidence-based filtering (407) is an ion count based empirical filtering, wherein preferably ion chromatograms are extracted for each precursor based on tolerances, preferably in terms of at least one of iRT, IM, and m/z, and for each extracted ion chromatogram (909), peak picking (907) is performed leading to precursor peak candidates, and for each of the precursor peak candidates, a spectrum centric score (908) is calculated based on how many of the fragment ions and precursor isotope ions match the MS2 and MS1 spectra respectively, and if none of the peak candidates passes a pre-specified threshold, then the precursor is dropped from further analysis.

8. The method according to any of the preceding claims, wherein at least one of the peptide centric search (411) and the second peptide centric search is carried out by using information from a spectral library (601) to analyse the data specifically for selected precursors only.

9. The method according to any of the preceding claims, wherein calibration (406) comprises determination of at least one parameter associated with a respective fragment: mass to charge ratio, retention time, in particular indexed retention time, expected fragment ion relative intensities, and ion mobility, and wherein the data, which is a set of DIA data (1002), is subjected to a spectrum centric analysis (1006) using a protein database (1003), from which precursors are identified (1007), and the parameters are adjusted by using a prediction model (1004) preferably based on the same protein database (1003) to generate a predicted library (1005) for the basis of the calibration.

10. The method according to any of the preceding claims, wherein calibration (406) comprises determination of at least one parameter associated with a respective fragment: mass to charge ratio, retention time, in particular indexed retention time, expected fragment ion relative intensities, and ion mobility, and wherein the data, which is a set of DIA data (1002), is subjected to a spectrum centric analysis (1006) using a protein database (1003), from which precursors are identified (1007), and the parameters are adjusted by using a prediction model (1004) preferably based on the same protein database (1003) to generate a predicted library (1005) for the basis of the calibration but using a specific selection (1010) for the calibration and using that selection for the peptide centric analysis (411, 1011).

11. Method according to any of the preceding claims, wherein the data (402) is in the form of a the sample mass spectroscopic intensity data acquired as a function of mass to charge ratio (m/z), of retention time (RT) as well as of ion mobility (IM) determined using an LC tandem mass spectroscopy method, preferably selected from the group of LC-MRM or LC-DIA.

12. Method according to any of the preceding claims, wherein the data (402) is a set of data independent acquisition data obtained from a sample (400) in an LC-MS/MS experiment and wherein the sample is a complex mixture of at least one protein of interest and further proteins and/or other biomolecules in the form of a complex native biological matrix which has been digested prior to LC-MS/MS analysis.

13. Method according to any of the preceding claims, wherein the at least one protein of interest is a protein based exclusively on proteinogenic amino acids, or is based on proteinogenic amino acids and carries post-translational modifications.

14. Use of a method according to any of the preceding claims for the determination of at least one of the composition of the sample including quantitative information about the constituents, or a medically relevant conformation of the constituents, for the determination or the influence of protein-based drugs, for the influence of drugs or other ligands on proteins, or for quality control of protein-based pharmaceutical preparations.

15. A computer program product to cause an LC-MS device to execute the steps of the method according to any of the preceding claims 1-13 or a computer-readable medium having stored thereon such a computer program product.
